# EUROPEAN PATENT SPECIFICATION

(11) **EP 1 945 234 B1**
(45) Date of publication and mention of the grant of the patent: **19.12.2012**
(21) Application number: 06798857.6
(22) Date of filing: 22.09.2006
(51) Int. Cl.: A61K 35/74

(54) **COMPOSITION FOR PREVENTING OR TREATING ARTHRITIS COMPRISING LACTIC ACID BACTERIA AND COLLANGEN AS ACTIVE INGREDIENTS**
ZUSAMMENSETZUNG ZUR PRÄVENTION ODER BEHANDLUNG VON ARTHRITIS MIT MILCHSÄURE-BAKTERIEN UND KOLLANGEN ALS WIRKSTOFFE
COMPOSITION POUR LA PRÉVENTION OU LE TRAITEMENT D'ARTHRITE COMPORTANT DES BACTÉRIES LACTIQUES ET DU COLLAGÈNE EN TANT QUE PRINCIPES ACTIFS

(30) Priority: 23.09.2005 KR 20050088465; 29.07.2006 KR 20060071796
(43) Date of publication of application: 23.07.2008
(73) Proprietor: Gwangju Institute of Science and Technology, Gwangju 500-712 (KR)
(72) Inventor: IM, Sin Hyeog Department of Life Science, Buk-gu Gwangju 500-712 (KR); SO, Jae Seon Department of Life Science, Buk-gu Gwangju 500-712 (KR); YI, Hwa Joong Department of Life Science, Buk-gu Gwangju 500-712 (KR)
(74) Representative: Viering, Jentschura & Partner
(86) International application number: PCT/KR2006/003774
(87) International publication number: WO 2007/035057

(56) References cited:
- EP-A- 1 354 590
- WO-A-2004/069178
- US-A1- 2005 147 645
- US-B1- 6 333 304
- US-B2- 6 585 993
- KATO I ET AL: "Suppressive effects of the oral administration of Lactobacillus casei on type ii collagen-induced arthritis in DBA/1 mice" LIFE SCIENCES 19980717 US, vol. 63, no. 8, 17 July 1998 (1998-07-17), pages 635-644, XP002572535 ISSN: 0024-3205
- HIROSHI KANO ET AL: "Oral administration of milk fermented with Lactobacillus delbrueckii ssp. bulgaricus OLL1073R-1 to DBA/1 mice inhibits secretion of proinflammatory cytokines" CYTOTECHNOLOGY, KLUWER ACADEMIC PUBLISHERS, DO, vol. 40, no. 1-3, 1 November 2002 (2002-11-01), pages 67-73, XP019236754 ISSN: 1573-0778
- GARCIA G ET AL: "Suppression of collagen-induced arthritis by oral or nasal administration of type II collagen" JOURNAL OF AUTOIMMUNITY 199911 GB, vol. 13, no. 3, November 1999 (1999-11), pages 315-324, XP002572536 ISSN: 0896-8411
- DATABASE WPI Week 200035 Thomson Scientific, London, GB; AN 2000-402970 XP002572543 & JP 2000 125810 A (YANGU KK) 9 May 2000 (2000-05-09)
- MCALINDON T E ET AL: "Glucosamine and chondroitin for treatment of osteoarthritis: A systematic quality assessment and meta-analysis" JOURNAL OF THE AMERICAN MEDICAL ASSOCIATION 20000315 US, vol. 283, no. 11, 15 March 2000 (2000-03-15), pages 1469-1475, XP002572537 ISSN: 0098-7484
- DEAL C L ET AL: "NUTRACEUTICALS AS THERAPEUTIC AGENTS IN OSTEOARTHRITIS THE ROLE OF GLUCOSAMINE, CHONDROITIN SULFATE, AND COLLAGEN HYDROLYSATE" RHEUMATIC DISEASES CLINICS OF NORTH AMERICA, W.B. SAUNDERS, PHILADELPHIA, PA, US, vol. 25, no. 2, 1 May 1999 (1999-05-01), pages 379-395, XP009050485 ISSN: 0889-857X
- KIM YOO KYUNG ET AL: "Anti-inflammation activity of Actinidia polygama." ARCHIVES OF PHARMACAL RESEARCH (SEOUL), vol. 26, no. 12, December 2003 (2003-12), pages 1061-1066, XP002572538 ISSN: 0253-6269
- KIM SUNG YONG ET AL: "Inhibitory effects of plant extracts on adjuvant-induced arthritis" ARCHIVES OF PHARMACAL RESEARCH (SEOUL), vol. 20, no. 4, 1997, pages 313-317, XP002572539 ISSN: 0253-6269
- DATABASE EMBASE [Online] ELSEVIER SCIENCE PUBLISHERS, AMSTERDAM, NL; 2000, GIL W ET AL: "Inhibitory effects of methanol extract of seeds of job's tears (coix lachryma-jobi l. Var. Ma-yuen) on nitric oxide and superoxide production in raw 264.7 Macrophages" XP002572540 Database accession no. EMB-2000278333 & IMMUNOPHARMACOLOGY AND IMMUNOTOXICOLOGY 2000 US, vol. 22, no. 3, 2000, pages 545-554, ISSN: 0892-3973
- DATABASE TCM -SIPO [Online] 9 May 2001 (2001-05-09), "A Chinese medicine Shexian Rheumatism Pill/A pharmaceutical composition for the treatment of rheumatic joint diseases and its preparation method" XP002572541 Database accession no. CN1293988 & CN 1 293 988 A (WANG ZHONGFU [CN]) 9 May 2001 (2001-05-09)
- DATABASE TCM -SIPO [Online] 10 August 2005 (2005-08-10), "A Chinese medicine preparation used for the treatment of rheumatic and rheumatoid arthritis / A Chinese medicine composition and its preparation method, which can be used for the treatment of rheumatic and rheumatoid arthritis" XP002572542 Database accession no. CN1651014 & CN 1 651 014 A (FENG ENPING [CN]) 10 August 2005 (2005-08-10)

## Description

### BACKGROUND OF THE INVENTION

### FIELD OF THE INVENTION

The present invention relates to a composition for preventing or treating arthritis.

### DESCRIPTION OF THE RELATED ART

Arthritis is one of the chronic diseases in which five percentages of worldwide human population are suffering. Its etiology involves inflammation at synovial membrane of articular capsule, which ultimately causes edema and pain in systemic joints. Arthritis is progressive degenerative disease and induces deformation and difficulties in bending of joints, and is very likely to produce serious effects if left untreated.

Little is known about the direct cause of arthritis. Various therapeutic drugs have been used for arthritis, including steroidal anti-inflammatory agents such as cortisone and other adrenocortical hormones, non-steroidal anti-inflammatory agents such as aspirin, piroxicam and indomethacin, anti-rheumatic agents such as chloroquinones and D-penicillamine, anti-gout agents such as colchicine, and immune suppressive agents such as cyclophosphamide, azathioprine, methotrexate and levamisol. However, these drugs are not considered to be fundamental therapeutics and the administration of steroidal drugs is somewhat restricted due to their side effects. Aspirin-based drugs used to relieve pain and remove inflammation associated with arthritis exhibit adverse effects against stomach; therefore, their continuous administration for treating arthritis is nearly impossible.

Since the conventional chemotherapeutic drugs described above have serious shortcomings including adverse effects, and limited anti-inflammation efficacy on developed arthritis, non-steroidal anti-inflammatory drug such as indomethacin is currently administered as an alternative therapeutic realm.

Accordingly, there is a long-felt need to develop novel therapeutics for arthritis showing better anti-inflammatory and pain relief efficacies together with overcoming drawbacks of conventional drugs described above. Since the prolonged administration of drugs is typically required for arthritis, the development of therapeutics with much less adverse effects is a significant consideration factor. Drugs administered via intravenous and intraperitoneal route are tedious to be administered and are likely to be accompanied with side effects such as allergy and shock. Therefore, drugs showing safety and convenience in administration are in demand.

Most of health foods for treating arthritis comprise cartilage constituents as a main ingredient. Korean Pat. Appln. No. 10-1997-0035934 discloses a therapeutic for arthritis comprising peptides derived from type II collagen, and Korean Pat. Appln. No. 10-2003-00433119 suggests a health food composition for treating degenerative arthritis comprising 40-45% of gluosamine, 30% of mucopolysaccharide protein, vitamin C, *Achyranthes japonica, Chaenomelis sinensis, Eucommia ulmoides* and shark cartilage extract.

As examples of drugs containing lactic acid bacteria, U.S. Pat. Appln. Publication No. 2005/007441 discloses that irritable bowel syndrome and inflammation could be alleviated by ingesting Bifidobacteria. In addition, Ehud Baharav et al. reported that the oral administration of live Lactobacillus GG or heat-inactivated Lactobacillus could prevent the development of arthritis (Ehud Baharav et al., Nutritional Immunology, 134:1964-1969(2004)). B. Sheil et al. revealed that *Lactobacillus salivarus* 118 administered via subcutaneous or oral route would alleviate irritable bowel syndrome and arthritis in arthritis model of mice. Given that the immunomodulatory action of Lactobacillus is exhibited in arthritis and bowel syndrome, it can be demonstrated that Lactobacillus modulates immune hypersensitivity and autoimmune response through regulating TGF-β level in T-lymphocytes (B Sheil et al., Gut, 53:694-700(2004)).

Yang et al. reported that peptides derived from type II collagen carrying T cell antigenic determinants are orally administered to collagen-induced arthritis of mouse models to prevent or treat collagen-induced arthritis (H. I. Yang, et al., J. of The Korean Rheumatism Association, 12:7(04)(2000)).

### DETAILED DESCRIPTION OF THIS INVETNION

The present inventors have made intensive studies to develop novel compositions for preventing or treating arthritis, having enhanced therapeutic efficacies and dramatically reduced adverse effects. As results, we have discovered that lactic acid bacteria administered to subjects together with collagen serving as self antigens for arthritis, greatly enhanced immunetolerance and suppressed joint-specific inflammation, and finally helped the successful prevention and treatment of arthritis.

Accordingly, it is an object of this invention to provide a composition for preventing or treating arthritis.

It is another object of this invention to provide a composition for use in preventing or treating arthritis.

Other objects and advantages of the present invention will become apparent from the following detailed description together with the appended claims and drawings.

In one aspect of this invention, there is provided a composition for preventing or treating arthritis, which comprises a lactic acid bacterium and collagen as active ingredients.

In still another aspect of this invention, there is provided a use of a composition comprising a lactic acid bacterium and collagen for use in a method for preventing or treating arthritis.

The present inventors have made intensive studies to develop novel compositions for preventing or treating arthritis, having enhanced therapeutic efficacies and dramatically reduced adverse effects. As results, we have discovered that lactic acid bacteria administered to subjects together with collagen serving as self antigens for arthritis, highly enhanced immunetolerance and suppressed joint-specific inflammation, and finally helped the successful prevention and treatment of arthritis.

In the composition of this invention, the active ingredient comprises lactic acid bacteria and collagen.

The lactic acid bacteria as an active ingredient have been reported to have therapeutic efficacies on intestinal disorders, *e.g*., alleviation of irritable bowel syndrome and improvement in intestinal function. The present invention is based on the novel findings of the present inventors in which lactic acid bacteria allows for the synergic increase in immunetolerance induced by collagen. In addition, the lactic acid bacteria in the present composition play a role in up-regulating the production of anti-inflammatory cytokines such as IL-10 and TGF-β.

The term used herein "lactic acid bacteria" refers to bacteria capable of producing lactic acid as main metabolites of carbohydrates, *e.g., Lactococcus, Lactobacillus, Leuconostoc, Propionibacterium, Enterococcus, Bifidobacterium, Streptococcus* and *Pediococcus* Preferably, the lactic acid bacterium suitable in this invention is at least one lactic acid bacterium selected from the group consisting of *Lactobacillus acidophilus, Lactobacillus casei, Lactobacillus gasseri, Lactobacillus delbrueckii, Lactobacillus fermentum, Lactobacillus bulgaricus, Lactobacillus helveticus, Streptococcus thermophilus, Streptococcus lactis, Enterococcus faecium, Enterococcus faecalis, Bifidobacterium bifidum, Bifidobacterium infantis, Bifidobacterium brave* and *Bifidobacterium longum.* Most preferably, the lactic acid bacterium suitable in this invention is *Lactobacillus casei.*

The preferable amount of lactic acid bacteria in the present composition is 10⁵ cfu-10¹¹ cfu per unit weight (g) of compositions.

Another active ingredient, collagen serves as self antigens to induce immunetolerance. Arthritis-specific drugs have not been successfully developed because self antigens involved in immune reactions are extremely diverse. The principle underlying the present invention is to employ immunetoierance elicited in the gut associated lymphoid tissue (GALT). Immunetolerance refers to immune reactions in which the immune system (GALT) present under epithelial cells of small intestine elicits specific immuno-suppressive reactions against materials or substances orally delivered. Collagen as an active ingredient suppresses selectively arthritis-specific inflammation through immunetolerance responses in the GALT.

Collagen used in this invention is type I or type II collagen, preferably, type II collagen. The preferable amount of collagen in the present composition is 0.1-2.0 wt%, more preferably, 0.2-1.5 wt%, most preferably 0.2-1.1 wt%.

According to a preferred embodiment, the composition of this invention further comprises glucosamine, chondroitin and their combination. All of collagen, glucosamine and chondroitin are cartilage constituents. Therefore, cartilage constituents (collagen) acting as self antigens permit to induce enhanced immunetolerance reactions. In addition, glucosamine allows promoting the formation and regeneration of cartilage. Chondroitin inhibits enzyme destructing cartilage thereby protect cartilage as well as induces immunetolerance responses.

In the present composition, glucosamine is preferably present in the form of D-(+)-glucosamine hydrochloride. In the present composition, chondroitin is preferably present in the form of chondroitin 6-sulfate salt. The preferable amount of glucosamine in the present composition is 1.0-4.0 wt%, more preferably 1.3-3.0 wt%, most preferably 2.0-3.0 wt%. The preferable amount of chondroitin in the present composition is 0.5-4.0 wt%, more preferably 0.7-3.0 wt%, most preferably 1.4-2.3 wt%.

According to a preferred embodiment, the composition of this invention further comprises a malt extract, Vitamin D and their combination.

Preferably, the malt extract is a fermented malt extract, more preferably, a malt extract containing about 2 wt% of chloride and 60-70 wt% of reducing sugars (e.g., maltose, sucrose and dextrose). The malt extract in the present composition allows suppression of inflammatory responses. The amount of malt extract in the present composition is preferably 10-70 wt%, more preferably 20-60 wt% and most preferably 35-55 wt%.

Vitamin D used in this invention is a iipophiiic Vitamin involved in bone metabolism and homeostasis of calcium phosphate. Preferably, Vitamin D used in this invention is Vitamin D₃. Vitamin D in the present composition promotes the productions of anti-inflammatory cytokine IL-10 and regulatory T cell (Tr1).

According to a preferred embodiment, the composition of this invention further comprises at least one extract selected from the group consisting of extracts of *Actinidia polygama,* cactus leaves, *Eucommia ulmoides,* seed of *Carthamus tinctorius, Coix lachrymal* and *Chaenomelis sinensis* The oriental medicines have been known to relieve pain and reduce inflammation. The amount of the oriental medicine in the present composition is preferably 5-50 wt%, more preferably 10-35 wt%, most preferably 20-35 wt%.

The present composition for preventing or treating arthritis may be prepared to provide a pharmaceutical composition by use of active ingredients and a pharmaceutically acceptable carrier. In the pharmaceutical compositions of this invention, the pharmaceutically acceptable carrier may be conventional one for formulation, including lactose, dextrose, sucrose, sorbitol, mannitol, starch, rubber arable, potassium phosphate, arginate, gelatin, potassium silicate, microcrystalline cellulose, polyvinylpyrrolidone, cellulose, water, syrups, methyl cellulose, methylhydroxy benzoate, propylhydroxy benzoate, talc, magnesium stearate, and mineral oils, but not limited to. The pharmaceutical composition according to the present invention may further include a lubricant, a humectant, a sweetener, a flavoring agent, an emulsifier, a suspending agent, and a preservative. Details of suitable pharmaceutically acceptable carriers and formulations can be found in Remington's Pharmaceutical Sciences (19th ed., 1995), which is incorporated herein by reference.

Since the pharmaceutical composition of this invention exerts its efficacies through immunetolerance, it should be administered via the oral route.

A suitable dose of the pharmaceutical composition of the present invention may vary depending on pharmaceutical formulation methods, administration methods, the patient's age, body weight, sex, severity of diseases, diet, administration time, administration route, an excretion rate and sensitivity for a used pharmaceutical composition. Physicians of ordinary skill in the art can determine an effective amount of the pharmaceutical composition for desired treatment. Preferably, the pharmaceutical composition of the present invention is administered with a daily dose of 0.01-2000 mg/kg (body weight).

According to the conventional techniques known to those skilled in the art, the pharmaceutical composition may be formulated with pharmaceutically acceptable carrier and/or vehicle as described above, finally providing several forms including a unit dose form and a multi-dose form. Non-limiting examples of the formulations include, but not limited to, a solution, a suspension or an emulsion in oil or aqueous medium, an extract, an elixir, a powder, a granule, a tablet and a capsule, and may further comprise a dispersion agent or a stabilizer.

The present composition for preventing or treating arthritis may be prepared to provide a food composition, in particular a health food composition. The food composition may comprise conventional additives for preparing food compositions, *e.g*., proteins, carbohydrates, lipids, nutritive substances and flavors. For example, where the food composition of this invention is provided as a drink, it may further comprise flavors and natural carbohydrates as well as lactic acid bacteria and collagen as active ingredients. Non-limiting examples of natural carbohydrates include, but not limited to, monosaccharide (*e.g*., glucose and fructose), disaccharide *(e.g.,* maltose and sucrose), oligosaccharide, polysaccharide *(e.g.,* dextrin and cyclodextrin) and sugar alcohol (e.g., xylitol, sorbitol and erythritol). Non-limiting examples of flavors include, but not limited to, natural flavors (*e.g.,* thaumatin and extract of *Stevia*) and synthetic flavors (*e.g.,* saccharin and aspartame). Considering availability to foods, the food composition of this invention is very useful in preventing or treating arthritis.

As demonstrated and illustrated in Examples, the composition of this invention induces immunetolerance by down-regulating the expression of proinflammatory cytokines such as TNF-α, IL-1β and IL-12, and up-regulating the expression of anti-inflammatory cytokines such as IL-10, Foxp3 and TGF-β, thereby enhancing Th2 type immune responses and reducing Th1 type immune responses. As results, the present invention suppresses dramatically arthritis-specific autoimmune responses and inflammation, leading to the successful prevention or treatment of arthritis.

The composition of this invention exerts excellent prevention or treatment efficacies on arthritis (osteoarthritis and rheumatoid arthritis), specifically rheumatoid arthritis which is one of inflammatory autoimmune diseases. In addition, since the active ingredients used in the composition of this invention have been recognized to be safe to human, the present composition is also considerably safe to human. Surprisingly, the present composition comprising active ingredients approved as raw materials of foods exhibits much better prevention or treatment efficacies than conventional chemical drugs (*e.g*., methotrexate).

According to conventional therapies for arthritis, there are limitations in the senses that self antigens involved in immune responses are extremely diverse and no arthritis-specific drugs can be developed. In addition, the conventional drugs for arthritis intend mostly to suppress only inflammation and their prolonged administration is very likely to induce adverse effects. Meanwhile, most of conventional health foods for arthritis comprise glucosamine, one of cartilage constituents, and extract of shark cartilage as main ingredients, thus possessing restricted efficacies. In contrast, the composition of this invention induces immunetolerance in the gut associated lymphoid tissue to suppress autoimmune responses and inflammation elicited specifically in joints, finally successfully preventing and treating arthritis.

### BRIEF DESCRIPTION OF THE DRAWINGS

Fig. 1 is a graph showing the analysis results using AIA (adjuvant induced arthritis) rats demonstrating the prevention efficacy of the present composition on arthritis. The composition of this invention decreased arthritis symptoms by 50% at the highest severity stage of arthritis. Clinical scores and paw thickness were measured.
Fig. 2 is a graph showing the analysis results using AIA rats demonstrating the treatment efficacy of the present composition on arthritis. The composition of this invention alleviated arthritis symptoms by 50% at the highest severity stage of arthritis. Clinical scores and paw thickness were measured.
Fig. 3 represents the comparative analysis results for the arthritis treatment efficacy of the present composition, each ingredient and conventional drug (methotrexate: MTX). It was evaluated that the present composition has a similar arthritis treatment efficacy to MTX.
Fig. 4 represents the arthritis treatment efficacy of the present composition compared with MTX. Clinical scores and paw thickness were measured. The results demonstrated that the present composition has a similar arthritis treatment efficacy to MTX.
Fig. 5 is a photograph showing the results of histological analysis to address the treatment efficacy of the present composition. Arrows indicate immune cells. The results represent that the present composition inhibits the infiltration of immune cells into joints. Such inhibition to infiltration occurred in a similar level to MTX.
Fig. 6 represents the results of real time PCR using mixed lymphocytes to reveal the effects of the present composition on the expression of proinflammatory cytokines.
Fig. 7 represents the results of real time PCR using CD4⁺ T cells to reveal the effects of the present composition on the expression of pro-and anti-inflammatory cytokines.
Fig. 8 represents the results of real time PCR using lymphocytes surrounding the joint to evaluate the effects of the present composition on the expression of pro-and anti-inflammatory cytokines.
Fig. 9 shows the results of cell proliferation assay using CD4⁺ T cells for the present composition.
Fig. 10 shows the results of cell proliferation assay using mixed lymphocytes surrounding the joint for the present composition.
Fig. 11 shows the results of ELISA to analyze the antibody concentrations altered by the present composition.

### EXAMPLES

### EXAMPLE 1: Preparation of Compositions of the Present Invention

Six oriental medicines (Gyeondong Market, Korea), 200 g of *Actinidia polygama,* 120 g of *Eucommia ulmoides,* 120 g of *Chaenomelis sinensis,* 120 g of *Coix lachryma,* 120 g of *seed of Carthamus tinctorius* and 120 g of cactus leaves were dried for 2 days at 75 °C and pulverized to form mixed powder. To this mixed powder, 60 g of glucosamine (Sigma), 50 g of chondroitin (Sigma), 25 g of type I collagen (Sigma), 1200 g of malt extract (Bacto), 36 mg of Vitamin D, 60 g of *Lactobacillus casei* in the form of a powder (Cell Biotech) were added and mixed, producing the exemplified immunomodulatory composition of this invention.

Daily dose for animal test in Lewis rats was determined to be 25 g (1.8 g of the exemplified composition of this invention and 23 g of feed powder). Feeds for rats were prepared by mixing 2160 g of the exemplified composition of this invention and 27600 g of feed powder.

Details of the exemplified composition of this invention are summarized in Table 1.

**TABLE 1**

| Ingredients | Amount | Amount ratio (wt%) |
|---|---|---|
| *Lactobacillus casei* | Approximately 7.5 x 10⁹ cfu (50 mg) | 2.7 |
| Glucosamine | 50 mg | 2.7 |
| Chondroitin | 40 mg | 2.2 |
| Collagen | 20 mg | 1.1 |
| Vitamin D | 30 µg | minute quantity |
| Malt extract | 1 g | 54.6 |
| *Actinidia polygama* | 170 mg | 9.29 |
| *Chaenomelis sinensis* | 100 mg | 5.5 |
| *Coix lachryma* | 100 mg | 5.5 |
| Seed of *Carthamus tinctorius* | 100 mg | 5.5 |
| Cactus leaves | 100 mg | 5.5 |
| *Eucommia ulmoides* | 100 mg | 5.5 |

### COMPARATIVE EXAMPLES 1-4: Preparation of Comparative Compositions Comprising Single Ingredient

The comparative compositions were prepared as Table 2.

**TABLE 2**

| Ingredients | Comp. Exam. 1 | Comp. Exam. 2 | Comp. Exam. 3 | Comp. Exam. 4 |
|---|---|---|---|---|
| *Lactobacillus casei* | + | - | - | - |
| Glucosamine | - | + | - | - |
| Chondroitin | - | + | - | - |
| Collagen | - | + | - | - |
| Malt extract | - | - | + | - |
| *Actinidia polygama* | - | - | - | + |
| *Chaenomelis sinensis* | - | - | - | + |
| *Coix lachryma* | - | - | - | + |
| Seed of *Carthamus tinctorius* | - | - | - | + |
| Cactus leaves | - | - | - | + |

### EXPERIMENTAL EXAMPLE 1: Analysis of Arthritis Prevention and Treatment Efficacy of the Present Composition

One animal model for rheumatoid arthritis is collagen-induced arthritis (CIA) induced by one of joint constituents, type II collagen, showing similarities to human rheumatoid arthritis in pathological clinical and immunological views. The animal model shows autoimmune diseases elicited by collagen-specific T cell and antibodies.

Another animal model for rheumatoid arthritis is for adjuvant-induced arthritis (AIA) induced by Mycobacterium tuberculosis germs rather than self antigens, exhibiting pathological symptoms and phenotypes of chronic arthritis. Even though molecules serving as self antigens have not yet reported, the animal model shows autoimmune diseases elicited by T cell-induced inflammation and tissue disruption in joint. This model shows similar symptoms to human spontaneous arthritis.

These two types of animal models were employed to accurately verify and confirm the efficacies of the present invention in the experimental examples, because causes and factors involved in the development of rheumatoid arthritis have not yet unraveled.

### Experimental Example 1-1: Generation of Collagen-Induced Arthritis Animal Models

Animal models for collagen-induced arthritis (CIA) were generated by injecting collagen involved in the development of rheumatoid arthritis as follows:
Four mg of chicken type II collagen (Sigma) were dissolved overnight at 4 °C in 1 ml of 100 mM acetic acid. The dissolved collagen was emulsified in 1 ml of Freund's complete adjuvant (Difco) containing 4 mg/ml of *Mycobacterium tuberculosis.* Lewis rat aged 6-8 weeks was immunized by the intradermal injection of 150 µl (300 µg) of the emulsion into the base of tail of rat. Separately, the dissolved collagen was emulsified in 1 ml of Freund's incomplete adjuvant (Difco). After 7 days of the primary immune response, 150 µl (300 µg) of the emulsion was intradermally injected into the base of tail of rat to induce the secondary immune (boosting) response. The symptoms of arthritis such as erythema and swelling could be observed 1-2 weeks after the secondary immune response.

### Experimental Example 1-2: Generation of Adjuvant-Induced Arthritis Animal Models

Animal models for adjuvant-induced arthritis (AIA) also exhibit pathological characteristics similar to human rheumatoid arthritis and are predominantly used in the art for animal tests along with coiiagen-induced arthritis animal models.

Ten mg of *Mycobacterium tuberculosis* were pulverized to give powder and mixed with 1 ml of Freund's incomplete adjuvant (Difco). Lewis rat aged 6-8 weeks was immunized by the intradermal injection of 100 µl (1 mg) of the mixture into the base of tail of rat. After 1 week of immunization, symptoms associated with arthritis appeared.

### Experimental Example 1-3: Analysis of Arthritis Prevention and Treatment Efficacy of the Present Composition

From the next day of the immunization, the compositions to be tested were orally administered everyday to the rats with a gastric feeding tube. Ten Lewis rats per group were selected. One group of adjuvant-induced arthritis animals was administered with a daily dose (25 g; 11.5 g/kg of the present composition) of mixtures containing animal feed powder and the present composition; the other group as a negative control consisted of adjuvant-induced arthritis animals administered with only animal feed powder. Following the administration, the severity of arthritis was evaluated by observing joint swelling, paw thickness and erythema, and the body weight of rats was measured.

For verifying the prevention efficacy on arthritis, AIA-inducing immunization was carried out 2 weeks after oral administration of the composition in Example 1. The experiment was carried out for total of 9 weeks. In addition, to reveal the treatment efficacy on arthritis, AIA-inducing primary immunization was initially performed and in turn the composition in Example 1 was orally administered. The experiment was also carried out for total of 9 weeks. Ten rats per group were employed. The appearance of each paw of rats such as paw thickness and erythema was observed and the clinical severity of arthritis was calculated according to scoring index of Tables 3 and 4.

**TABLE 3. Scoring Index in CIA models**

| Score | Disease progression |
|---|---|
| 0 | No appearance of erythema and swelling |
| 1 | Mild erythema and swelling observed only at the central portion of foot or ankle joint |
| 2 | Mild erythema and swelling observed from ankle joint to the central portion of foot |
| 3 | Moderate erythema and swelling observed from ankle joint to metatarsal bone joint |
| 4 | Marked erythema and swelling observed on ankle, foot and toe |

**TABLE 4. Scoring Index in AIA models**

| Score | Disease progression |
|---|---|
| 0 | No appearance of erythema and swelling |
| I | Mild erythema or swelling observed on either toe or finger |
| 2 | Moderate erythema or swelling observed on toe and/or finger |
| 3 | Moderate erythema or swelling observed on either ankle or wrist |
| 4 | Marked erythema and swelling observed on toe or finger and ankle or wrist, no bending of ankle or wrist |

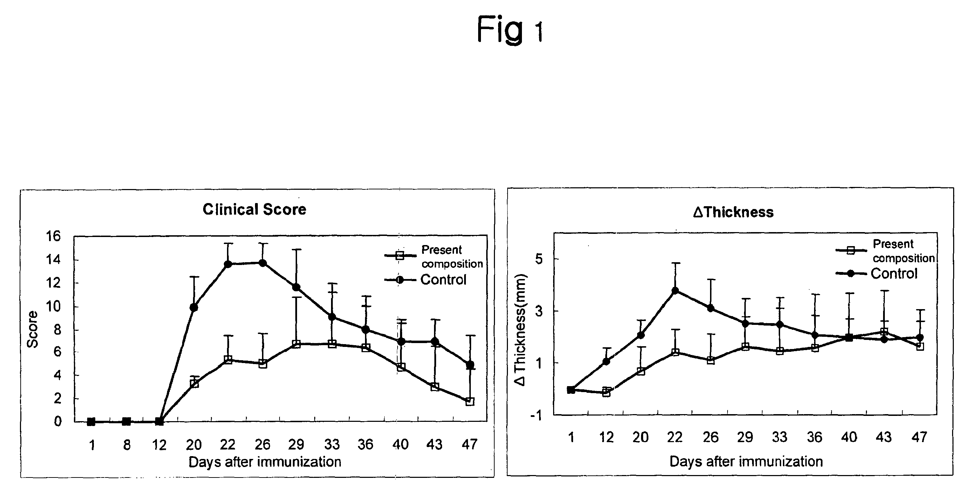

As shown in the clinical score of Fig. 1 representing the results of arthritis prevention test, the control group as adjuvant-induced arthritis model was observed to show arthritis symptoms 2 weeks after immunization, and the severity of arthritis was reduced gradually 5 weeks after immunization. Unlikely, the group administered with the present composition exhibits significantly reduced severity of arthritis. The control group exhibits the swelling of paws from 2 weeks after the immunization, as shown in Fig. 1. Unlikely, the group administered with the present composition shows considerably reduced swelling of paws. Therefore, it could be appreciated that the composition of this invention has excellent prevention efficacy on arthritis.

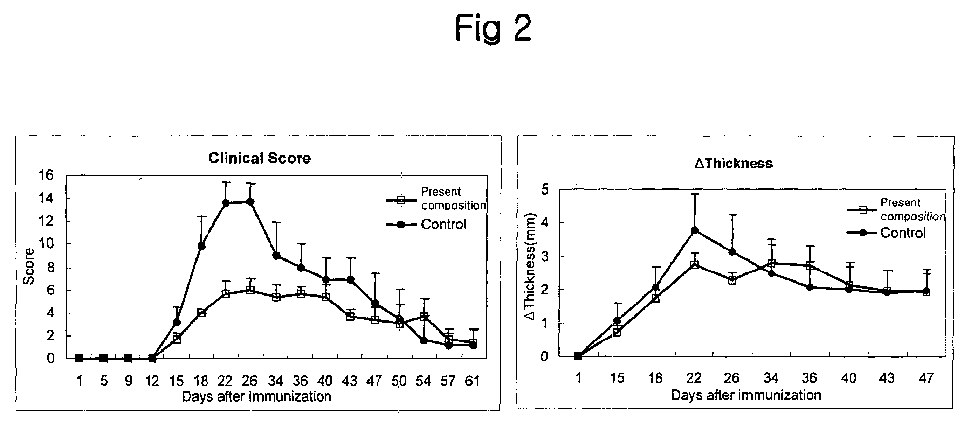

As shown in the clinical score of Fig. 2 representing the results of arthritis treatment test, the control group as adjuvant-induced arthritis model was observed to show arthritis symptoms 2 weeks after immunization, and the severity of arthritis was reduced gradually 5 weeks after immunization. Unlikely, the group administered with the present composition exhibited significantly reduced severity of arthritis. The control group exhibited the swelling of paws from 2 weeks after the immunization, as shown in Fig. 1. Unlikely, the group administered with the present composition showed considerably reduced swelling of paws. Therefore, it could be understood that the composition of this invention has excellent treatment efficacy on arthritis.

### EXPERIMENTAL EXAMPLE 2: Comparative Tests for Treatment Efficacy on Arthritis

The composition of Example 1 revealed to have considerable efficacies on arthritis, hence more accurate and reliable tests were carried out to confirm the prevention and treatment efficacies on arthritis.

The treatment efficacy of compositions of this invention (in particular, composition of Example 1) was compared with that of other compositions as indicated in Comparative Examples of Table 2. The experimental procedures are similar to Experimental Example 1-3. Each group consists of 10 rats.

The control group is the non-administered group and MTX group is administered twice a week with 60 µg unit dosage of a conventional drug for arthritis, methotrexate. Oriental medicine group is administered with a mixed extract of *Actinidia polygama, Eucommia ulmoides,* cactus leaves, seed of *Carthamus tinctorius, Coix lachryma* and *Chaenomelis sinensis.* Joint constituent group is administered with a mixture of glucosamine, collagen and chondroitin. Present composition group is administered with the present composition of Example 1. Each composition was orally administered for 2 week and the primary immunization was induced using collagen. One week later, the secondary immunization was induced and the clinical score and paw thickness were measured.

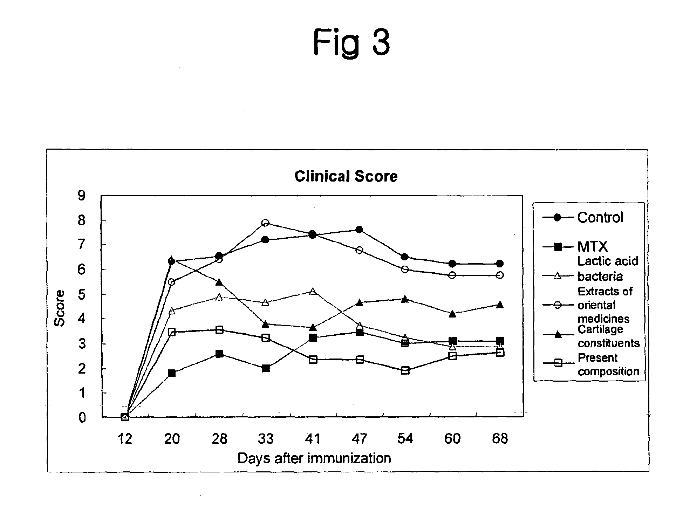

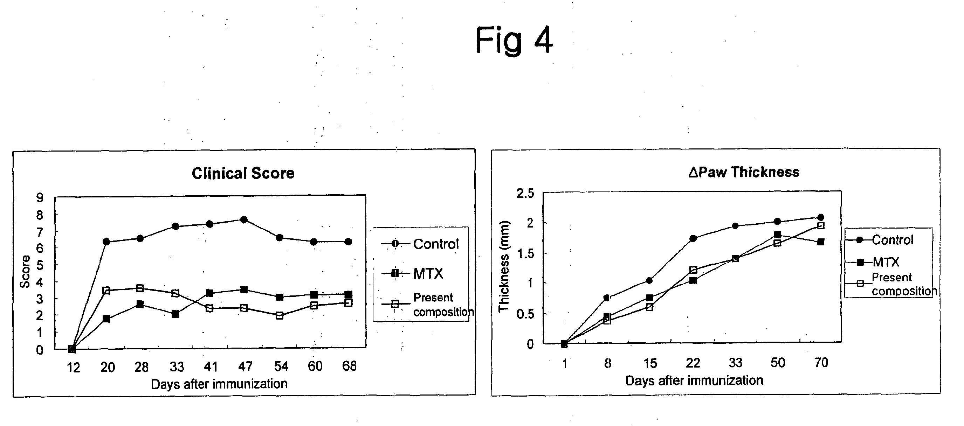

As shown in Figs. 3 and 4, symptoms associated with arthritis appeared after 20 days of CIA-induced immunization. The group administered with mixed oriental medicine showed little or no treatment efficacy. In the group administered with lactic acid bacteria, the treatment efficacy was low during acute phase. Although the treatment by MTX was reveaied to be better until acute phase (until 35 days after immunization) than the present composition, the present composition exhibited better treatment efficacy during chronic phase than MTX (see Figs. 3 and 4).

Accordingly, these results clearly show that the compositions of this invention give similar or improved treatment efficacy on arthritis compared with MTX. Moreover, the enhance treatment efficacy of the present composition is suggested to be ascribed to the synergic contribution of lactic acid bacteria to immunetolerance responses induced by collagen, leading to the synergic increase in immunetolerance responses.

### EXPERIMENTAL EXAMPLE 3: Histological Analysis for Treatment Efficacy on Arthritis

The experiment for treatment efficacy on arthritis was carried out as Experimental Example 2. After 35 days of arthritis-inducing immunization, rats having the mean clinical score were sacrificed and their swelled ankle joint were histologically analyzed. Joints dissected were washed with PBS and fixed for 24 hr in 4% paraformaldehyde, followed by decalcification for 2-3 days in decalcification solution (Merck). The decalcified joint tissues were embedded in paraffin, sectioned to 0.6-1 µm, deparaffinized, stained with hematoxylin and eosin, and observed under microscope. A basic dye, hematoxylin binds to nucleus containing negative-charged DNA and RNA molecules and an acidic dye, eosin binds to cytoplasmic proteins and collagen with positive charge.

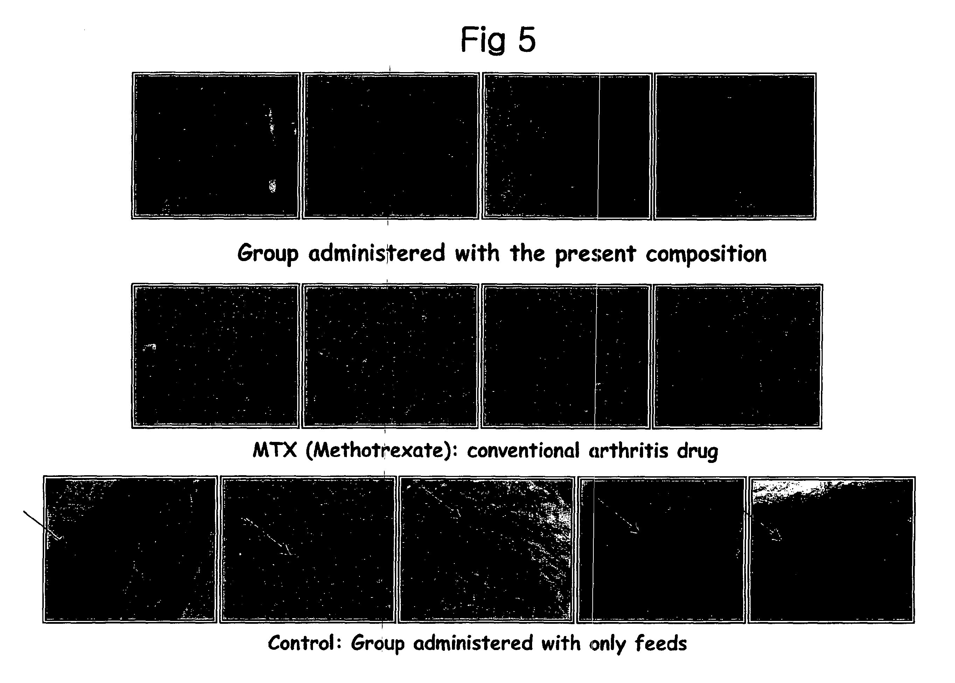

As shown in Fig. 5, it was observed in control CIA animal model that numerous lymphocytes infiltrate into tissues. Lymphocytes were stained with hematoxylin and seen as blue dots, while surrounding joint tissues were stained red with eosin. In contrast, there was little or no occurrence of the infiltration of lymphocytes for the group administered with either the present composition or MTX.

Accordingly, it would be recognized that the composition of this invention inhibits effectively lymphocyte-induced immune inflammation.

### EXPERIMENTAL EXAMPLE 4: Analysis of Effects on the Expression of Proinflammatory Cytokines (Using Mixed Lymphocytes)

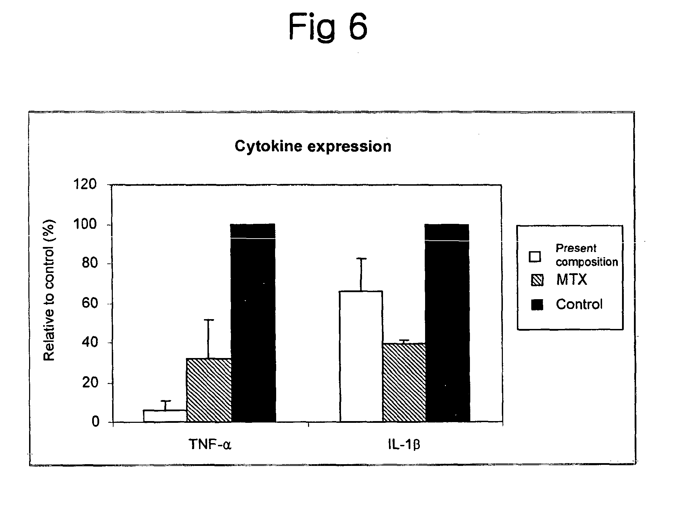

The experiment for treatment efficacy on arthritis was carried out as Experimental Example 2. After 35 days of arthritis-inducing immunization, rats having the mean clinical score were sacrificed and their spleen and draining lymph nodes were dissected, which were ground to give mixed lymphocytes. The mixed lymphocytes (5 x 10⁶ cells/well) were aliquoted into 24-well plate and stimulated for 6 hr with 20 µg/well of type II collagen. Then, cells were harvested and treated with Trizol to yield the total RNAs. From 1 µg RNA of each group, cDNA was synthesized using oligo dT primer and reverse transcriptase (Promega). The quantitative real-time PCR was carried out using the synthesized cDNA as template, 10 pmol primers for cytokines and SYBR premix to analyze and compare the levels of cytokine expression (Fig. 6). The quantitative real-time PCR reactions were conducted under the following thermal conditions: 10 min at 94°C followed by 40 cycles of 30 sec at 94°C, 30 sec at 62°C and 30 sec at 72°C. The quantification was performed at every cycle. The primers used for cytokine are: β-actin, 5'-TAC TGC CCT GGC TCC TAG CA-3' (forward primer) and 5'-TGG ACA GTG AGG CCA GGA TAG (reverse primer); TNF-α, 5'-CAC GTC GTA GCA AAC C-3' (forward primer) and 5'-GGT GAG GAG CAC ATA GT-3' (reverse primer); and IL-1β, 5'-GGG TTC CAT GGT GAA GTC AAC-3' (forward primer) and 5'-CAC CTC TCA AGC AGA GCA CAG-3' (reverse primer).

The results were normalized to the expression of the β-actin gene as a housekeeping gene. The data indicated in Fig. 6 are a relative value to the control (100%). The expression level of the proinflammatory cytokine, TNF-α was analyzed to be the lowest in the group administered with the present composition. Another proinflammatory cytokine, IL-1β was also expressed in a lower level in the group administered with the present composition.

Accordingly, our invention shows that the composition of this invention inhibits the expression of proinflammatory cytokines to suppress inflammation reactions, leading to the treatment of arthritis.

### EXPERIMENTAL EXAMPLE 5: Analysis of Effects on the Expression of Proinflammatory Cytokines (Using CD4⁺ T Cells)

Spleen from normal rats were dissected, ground and treated for 30 min with 25 µg/ml mitomycin C (Sigma) to obtain splenocytes as APC (antigen presenting cell). 10⁵ cells/well of the splenocytes were aliquoted into 24-well plates and stimulated with 20 µg/well of type II collagen for 30 min.

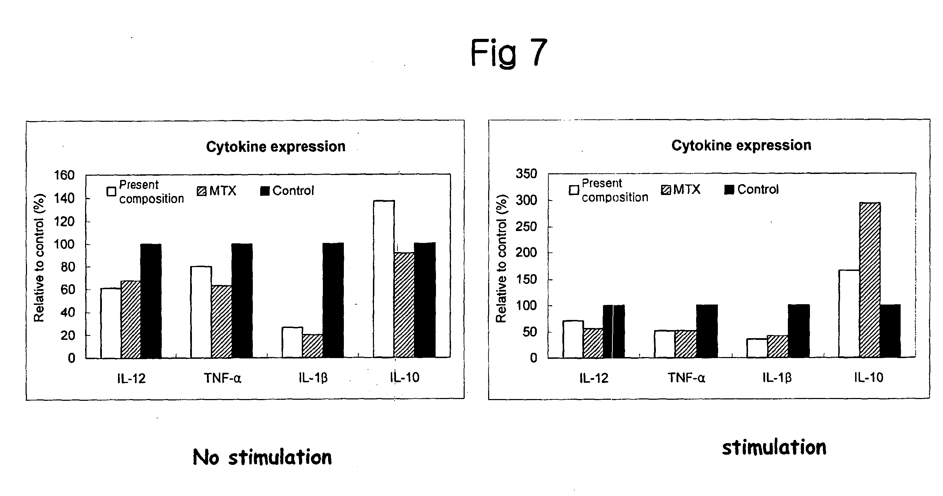

The experiment for treatment efficacy on arthritis was carried out as Experimental Example 2. After 70 days of arthritis-inducing immunization, rats having the mean clinical score were sacrificed and their draining lymph nodes were dissected, which were ground to give mixed lymphocytes. From the mixed lymphocytes, CD4⁺ T cells were separated using CD4⁺ magnetic beads (Miltenyi Biotec) and their aliquots (5 x 10⁶ cells/well) were transferred into the 24-well plates containing APC and collagen to stimulate for 24 hr. Then, cells were harvested and treated with Trizol to yield a total RNA. From 1 µg RNA of each group, cDNA was synthesized using oligo dT primer and reverse transcriptase (Promega). The quantitative real-time PCR was carried out using the synthesized cDNA as template, 10 pmol primers for cytokines and SYBR premix to analyze and compare the levels of cytokine expression (Fig. 7). The quantitative real-time PCR reactions were conducted under the following thermal conditions: 10 min at 94°C followed by 40 cycles of 30 sec at 95°C, 30 sec at 62°C and 30 sec at 72°C. The quantification was performed at every cycle. The primers used for cytokine are: β-actin, 5'-TAC TGC CCT GGC TCC TAG CA-3' (forward primer) and 5'-TGG ACA GTG AGG CCA GGA TAG (reverse primer); IL-12, 5'-GGT CAC ACT GAA CCA AAG (forward primer) and 5'-GTG GGT CCG GTT TGA T (reverse primer); TNF-α, 5'-CAC GTC GTA GCA AAC C-3' (forward primer) and 5'-GGT GAG GAG CAC ATA GT-3' (reverse primer); IL-1β, 5'-GGG TTC CAT GGT GAA GTC AAC-3' (forward primer) and 5'-CAC CTC TCA AGC AGA GCA CAG-3' (reverse primer); and IL-10, 5'-ACC AGC TGG ACA ACA TAG TGC (forward primer) and 5'-ATC CAG AGG GTC TTC AGC TTC (reverse primer).

The results were normalized to the expression of the β-actin gene as a housekeeping gene. The data indicated in Fig. 7 are a relative vaiue to the control (100%). Comparing the results from samples stimulated with collagen and unstimulated samples, the expression levels of the proinflammatory cytokines, IL-12, TNF-α and IL-1β were elucidated to be low in the group administered with the present composition. In particular, the present composition significantly reduced the expression level of IL-1β in stimulated CD4⁺ T cells to 50% of that of control. A Th2 cytokine, IL-10 capable of suppressing Th1-mediated inflammation was expressed in higher levels in the group administered with the present composition than the control.

On the basis of results of cytokine expressions in CD4⁺ T cells, which play a pivotal role in inflammation reactions, it can be concluded that the composition of the present invention enhances the expressions of anti-inflammatory cytokines as well as inhibits the expressions of proinflammatory cytokines.

### EXPERIMENTAL EXAMPLE 6: Analysis of Effects on the Expression of Pro-and Anti-inflammatory Cytokines (Using Lymphocytes Surrounding Joints)

The experiment for treatment efficacy on arthritis was carried out as Experimental Example 2. After 75 days of arthritis-inducing immunization, rats having the mean clinical score were sacrificed and their popliteal lymph nodes and inguinal lymph nodes surrounding joints were dissected, which were ground to give mixed lymphocytes.

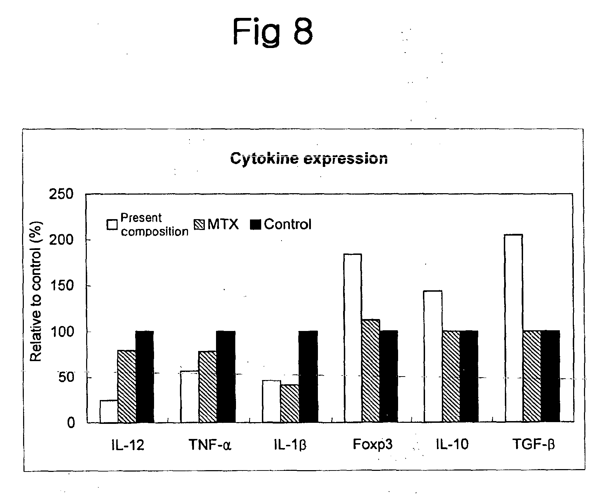

The mixed lymphocytes (5 x 10⁶ cells/well) were aliquoted into 24-well plate and stimulated for 24 hr with 40 µg/well of type II collagen. Then, cells were harvested and treated with Trizol to yield the total RNAs. From 1 µg RNA of each group, cDNA was synthesized using oligo dT primer and reverse transcriptase (Promega). The quantitative real-time PCR was carried out using the synthesized cDNA as template, 10 pmol primers for cytokines and SYBR premix to analyze and compare the levels of cytokine expression (Fig. 8). The quantitative real-time PCR reactions were conducted under the following thermal conditions: 10 min at 94°C followed by 40 cycles of 30 sec at 95°C, 30 sec at 62°C and 30 sec at 72°C. The quantification was performed at every cycle.

The results were normalized to the expression of the β-actin gene as a housekeeping gene. The data indicated in Fig. 8 are a relative value to the control (100%). It was revealed that the expression levels of proinflammatory cytokines, IL-12, TNF-α and IL-1β were lower in the group administered with the present composition as CD4⁺ T cell-using experiments. In particular, the present composition significantly reduced the expression levels of IL-12 and IL-1β to less than 50% of that of control. Furthermore, the expressions of Foxp3, IL-10 and TGC-β were up-regulated by the present composition compared with the control.

Accordingly, these results lead us to reason that the present composition induces oral immunetolerance in lymphocytes surrounding joints. Interestingly, the present invention up-regulated the expression of the regulatory T cell marker Foxp3 as well as the expression of TGF-β, which is capable of suppressing inflammation.

Taken together, our works suggest that the composition of the present invention induces oral immunotolerance in lymph nodes surrounding joints directly involving the development of arthritis, which results in the up-regulation of expressions of anti-inflammatory cytokines such as Foxp3, IL-10 and TGF-β as well as the down-regulation of expressions of proinflammatory cytokines such as IL-12, TNF-α and IL-1β.

### EXPERIMENTAL EXAMPLE 7: Analysis of Cell Proliferation (Using CD4⁺ T Cells)

Spleen from normal rats were dissected, ground and treated for 30 min with 25 µg/ml mitomycin C (Sigma) to obtain splenocytes as APC (antigen presenting cell). 10⁵ cells/well of the splenocytes were aliquoted into 96-well plates and stimulated with 20 µg/well of type II collagen for 30 min.

The experiment for treatment efficacy on arthritis was carried out as Experimental Example 2. After 70 days of arthritis-inducing immunization, rats having the mean clinical score were sacrificed and their draining lymph nodes were dissected, which were ground to give mixed lymphocytes.

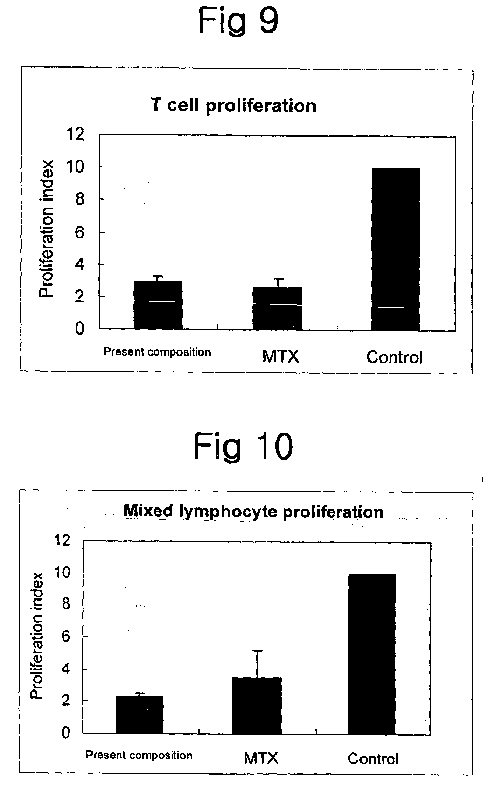

From the mixed lymphocytes, CD4⁺ T cells were separated using CD4⁺ magnetic beads (Miltenyi Biotec) and their aliquots (10⁵ cells/well) were transferred into the 94-well plates containing APC and collagen to stimulate for 56 hr. Then, cells were pulsed with 0.5 µCi [³H] thymidine (Perkin Elmer) for 16 hr and the thymindine incorporation was measured (Fig. 9). The data indicated in Fig. 9 are a relative value to the control (10).

In the case that arthritis is actively developed, the levels of the thymindine incorporation are measured to be higher because CD4⁺ T cells responding to arthritis antigens proliferate rapidly. In other words, the thymindine incorporation becomes higher as the severity of arthritis become larger. As represented in Fig. 9, the group administered with the present composition exhibits much lowered thymindine incorporation than that of the control group and showed similar results of MTX-treated group.

Accordingly, it could be concluded that CD4⁺ T cells in the group administered with the present composition lowered immune responses to collagen, demonstrating that the composition of the present invention confers immunetolerance to CD4⁺ T cells.

### EXPERIMENTAL EXAMPLE 8: Analysis of Cell Proliferation (Using Mixed Lymphocytes Surrounding Joints)

The experiment for treatment efficacy on arthritis was carried out as Experimental Example 2. After 75 days of arthritis-inducing immunization, rats having the mean clinical score were sacrificed and their popliteal lymph nodes and inguinal lymph nodes surrounding joints were dissected, which were ground to give mixed lymphocytes. The mixed lymphocytes (2 x 10⁵ cells/well) were aliquoted into 96-well plate and stimulated for 56 hr with 40 µg/well of type II collagen.

Then, cells were pulsed with 0.5 µCi [³H] thymidine (Perkin Elmer) for 16 hr and the thymindine incorporation was measured (Fig. 10). The data indicated in Fig. 10 are a relative value to the control (10).

As represented in Fig. 10, the group administered with the present composition exhibits the lowest thymindine incorporation. These results lead us to conclude that the composition of this invention induces oral immunetolerance in lymph nodes surrounding joints associated directly with arthritis, thereby greatly suppressing immune responses to collagen.

### EXPERIMENTAL EXAMPLE 9: Measurement of Production of Antibodies and IgG Isotypes to Collagen: ELISA Measurement

The experiment for treatment efficacy on arthritis was carried out as Experimental Example 2. After 35 days and 70 days of arthritis-inducing immunization, whole blood samples were prepared from rats having the mean clinical score and their serum components were separated for ELISA.

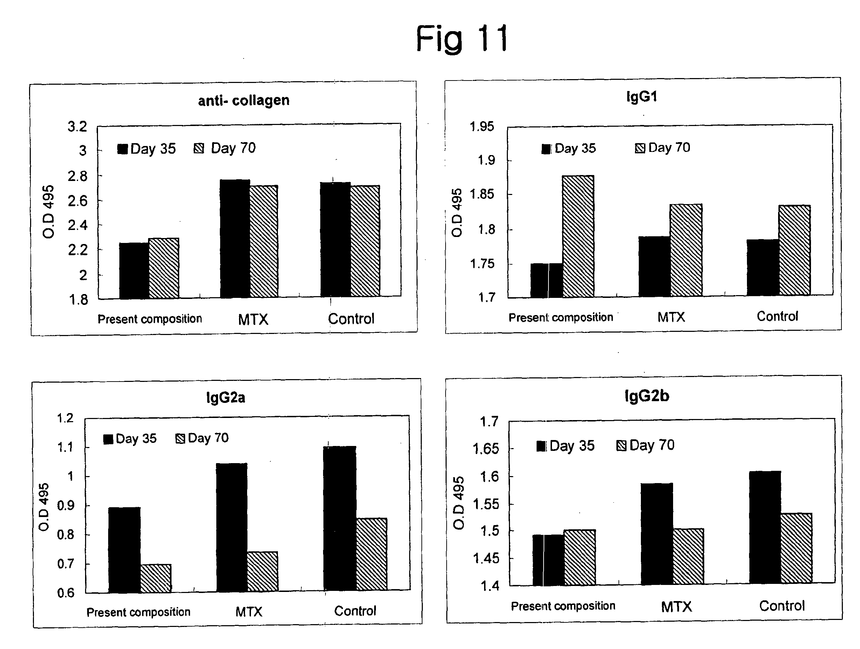

First, we measured the concentration of anti-collagen Ab present in serum samples. 100 µl/well of chicken type II collagen (1 µg/ml) aliquoted into 96-well plates were incubated for 12 hr at 4°C, and incubated with 100 µl of 1/10000 diluted serum of each group for 1 hr at 37°C, followed by incubating with 100 µl of anti-rat secondary Ab for 1 hr at 37°C. The color development was carried out using OPD and its absorbance was measured at 495 nm. As shown in Fig. 11, the group administered with the present composition exhibited the lowest concentration of anti-collagen Ab after 35 days and 70 days of immunization.

The IgG isotyping was conducted using anti-Ab against each IgG isotype (Immunology Consultant Laboratory). Various isotypes of IgGs such as IgG1, IgG2a, IgG2b and IgG2c have been reported and their analysis permits to reveal whether disease conditions are induced by Th1 or Th2 immune reactions. IgG1 is related to Th2 type immune reactions and IgG2 to Th1 type immune reactions. 100 µl/well of anti-Ab (2 µg/ml) to each IgG isotype aliquoted into 96-well plates were incubated for 12 hr at 4°C, and incubated with 100 µl of diluted serum of each group for 1 hr at 37°C, followed by incubating with 100 µl of anti-rat secondary Ab for 1 hr at 37°C. The color development was carried out using OPD and its absorbance was measured at 495 nm. As shown in Fig. 11, although the group administered with the present composition exhibited the lowest level of IgG1 on 35 days of immunization, it showed the highest level of IgG1 on 70 days of immunization. These results correspond to the increase in the expression of Th2 type cytokine IL-10 analyzed by real-time PCR, indicating that the composition of this invention suppresses Th1-mediated immune reactions through Th2 type immune reactions.

In contrast, the group administered with the present composition exhibited the lowest levels of IgG2a and IgG2b on both 35 days and 70 days of immunization. These results correspond to the down-regulation of proinflammatory cytokines such as TNF-α, IFN-γ and IL-1β by the present composition, addressing that the composition of this invention suppresses Th1 type immune reactions.

According to conventional therapies for arthritis, there are limitations in the senses that self antigens involved in immune reactions are extremely diverse and no arthritis-specific drugs can be developed. In addition, the convention drugs for arthritis intend mostly to suppress only inflammation and their prolonged administration is very likely to induce adverse effects. Meanwhile, most of conventional health foods for arthritis comprise glucosamine, one of cartilage constituents, and extract of shark cartilage as main ingredients, thereby possessing restricted efficacies. In contrast, the composition of this invention induces immunotolerance in the gut associated lymphoid tissue to suppress autoimmune reactions and inflammation generated specifically in joints, finally successfully preventing and treating arthritis.

## Claims

1. A composition comprising a lactic acid bacterium and collagen for use in a method for preventing or treating arthritis comprising administering the lactic acid bacterium and the collagen simultaneously to a subject.

2. The composition for use according to claim 1, wherein the composition further comprises glucosamine, chondroitin, or a combination thereof.

3. The composition for use according to claim 1, wherein the composition further comprises a malt extract, Vitamin D, or a combination thereof.

4. The composition for use according to claim 1, wherein the composition further comprises at least one extract selected from the group consisting of an extract of *Actinidia polygama,* an extract of cactus leaves, an extract of *Eucommia ulmoides,* an extract of seed of *Carthamus tinctorius,* an extract of *Coix lachrymal,* and an extract of *Chaenomelis sinensis.*

5. The composition for use according to claim 1, wherein the lactic acid bacterium is at least one lactic acid bacterium selected from the group consisting of *Lactobacillus acidophilus, Lactobacillus casei, Lactobacillus gasseri, Lactobacillus delbrueckii, Lactobacillus fermentum, Lactobacillus bulgaricus, Lactobacillus helveticus, Streptococcus thermophilus, Streptococcus lactis, Enterococcus faecium, Enterococcus faecalis, Bifidobacterium bifidum, Bifidobacterium infantis, Bifidobacterium brave* and *Bifidobacterium longum.*

6. The composition for use according to claim 5, wherein the lactic acid bacterium is *Lactobacillus casei.*

## Patentansprüche

1. Eine Zusammensetzung umfassend ein Milchsäurebakterium und Kollagen zur Verwendung in einem Verfahren zur Prävention oder Behandlung von Arthritis umfassend das gleichzeitige Verabreichen des Milchsäurebakteriums und des Kollagens an ein Individuum.

2. Die Zusammensetzung zur Verwendung nach Anspruch 1, wobei die Zusammensetzung ferner Glucosamin, Chondroitin oder eine Kombination davon umfasst.

3. Die Zusammensetzung zur Verwendung nach Anspruch 1, wobei die Zusammensetzung ferner einen Malzextrakt, Vitamin D oder eine Kombination davon umfasst.

4. Die Zusammensetzung zur Verwendung nach Anspruch 1, wobei die Zusammensetzung ferner mindestens einen Extrakt umfasst, der aus der Gruppe bestehend aus einem Extrakt von *Actinidia polygama,* einem Extrakt von Kaktusblättern, einem Extrakt von *Eucommia ulmoides,* einem Extrakt von *Carthamus tinctorius* Samen, einem Extrakt von *Coix lachrymal* und einem Extrakt von *Chaenomelis sinensis* ausgewählt ist.

5. Die Zusammensetzung zur Verwendung nach Anspruch 1, wobei das Milchsäurebakterium mindestens ein Milchsäurebakterium umfasst, das aus der Gruppe bestehend aus *Lactobacillus acidophilus, Lactobacillus casei, Lactobacillus gasseri, Lactobacillus delbrueckii, Lactobacillus fermentum, Lactobacillus bulgaricus, Lactobacillus helveticus, Streptococcus thermophilus, Streptococcus lactis, Enterococcus faecium, Enterococcus faecalis, Bifidobacterium bifidum, Bifidobacterium infantis, Bifidobacterium brave* und *Bifidobacterium longum* ausgewählt ist.

6. Die Zusammensetzung zur Verwendung nach Anspruch 5, wobei das Milchsäurebakterium *Lactobacillus casei* ist.

## Revendications

1. Composition comprenant une bactérie d'acide lactique et du collagène destinée à être utilisée dans un procédé de prévention ou de traitement de l'arthrite, qui consiste à administrer la bactérie d'acide lactique et le collagène simultanément à un sujet.

2. Composition destinée à être utilisée selon la revendication 1, la composition comprenant en outre de la glucosamine, de la chondroïtine, ou une combinaison de celles-ci.

3. Composition destinée à être utilisée selon la revendication 1, la composition comprenant en outre un extrait de malt, de la vitamine D, ou une combinaison de ceux-ci.

4. Composition destinée à être utilisée selon la revendication 1, la composition comprenant en outre au moins un extrait sélectionné dans le groupe consistant en un extrait *d'Actinidia polygama,* un extrait de feuilles de cactus, un extrait d'*Eucommia ulmoides,* un extrait de graine de *Carthamus tinctorius,* un extrait de *Coix lacrymal,* et un extrait de *Chaenomelis sinensis.*

5. Composition destinée à être utilisée selon la revendication 1, dans laquelle la bactérie d'acide lactique est au moins une bactérie d'acide lactique sélectionnée dans le groupe consistant en *Lactobacillus acidophilus, Lactobacillus casei, Lactobacillus gasseri, Lactobacillus delbrueckii, Lactobacillus fermentum, Lactobacillus bulgaricus, Lactobacillus helveticus, Streptococcus thermophilus, Streptococcus lactis, Enterococcus faecium, Enterococcus faecalis, Bifidobacterium bifidum, Bifidobacterium infantis, Bifidobacterium brave* et *Bifidobacterium longum.*

6. Composition destinée à être utilisée selon la revendication 5, dans laquelle la bactérie d'acide lactique est le *Lactobacillus casei.*
